# EUROPEAN PATENT APPLICATION

(11) **EP 1 639 952 A1**
(43) Date of publication of application: **29.03.2006**
(21) Application number: 04733483.4
(22) Date of filing: 17.05.2004
(51) Int. Cl.: A61B 17/56

(54) **ORTHOPEDIC INSTRUMENT**

(30) Priority: 24.06.2003 JP 2003178917
(71) Applicant: Nihon University, Tokyo 102-8275 (JP)
(72) Inventor: IWANARI, Shinkichi, c/o Nihon University, Chiyoda-ku, Tokyo 102-8275 (JP)
(74) Representative: Piésold, Alexander James
(86) International application number: PCT/JP2004/007002
(87) International publication number: WO 2004/112624

(57) **Abstract**

To provide an orthopedic apparatus with which a surgeon can perform a trimming operation safely without damaging blood vessels and nerves in the vicinity of an angle of jaw and without the need to make a large incision and a surgeon can perform a trimming operation by one hand with a smaller number of persons required for the operation.

The orthopedic apparatus has: a shank (11); a fan-shaped member (12) which is provided at the front end of the shank (11); and a coupling (13) which is provided at the rear end of the shank and to be connected to a driving source. The fan-shaped member (12) is inclined with respect to an extension (L) of the shank (inclination angle θ) ; file ridges (14) are formed on the fan-shaped member (12)'s surface (reverse side) which is oriented toward the rear end of the shank; and the shank (11) is constructed so as to rotate into a clockwise direction and a counterclockwise direction continuously.

## Description

### Technical Field

The present invention relates to improvement of an orthopedic apparatus such as a file which is used to remove burrs generated on cut surfaces of bones or the like.

### Background Art

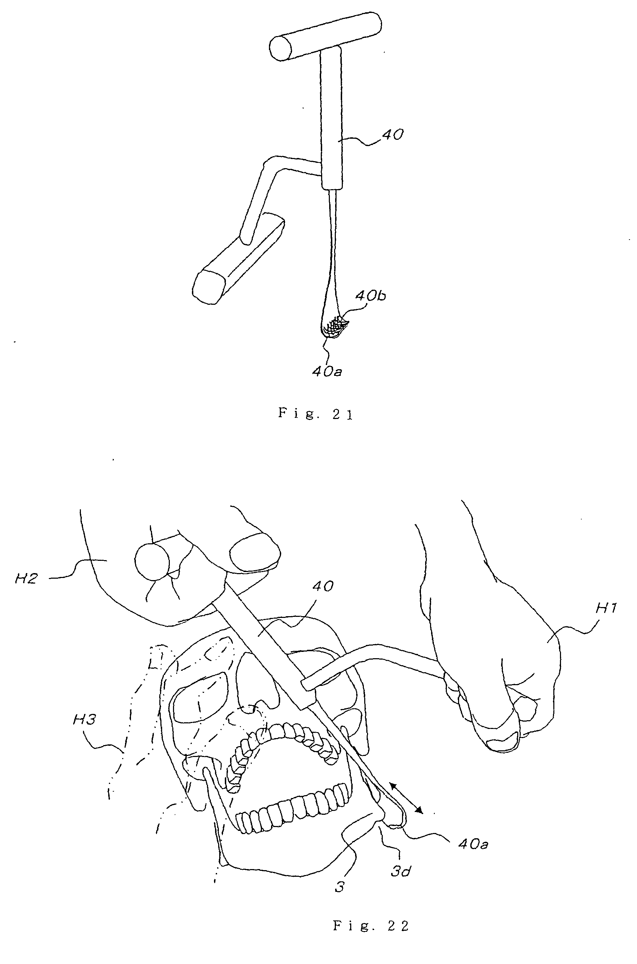

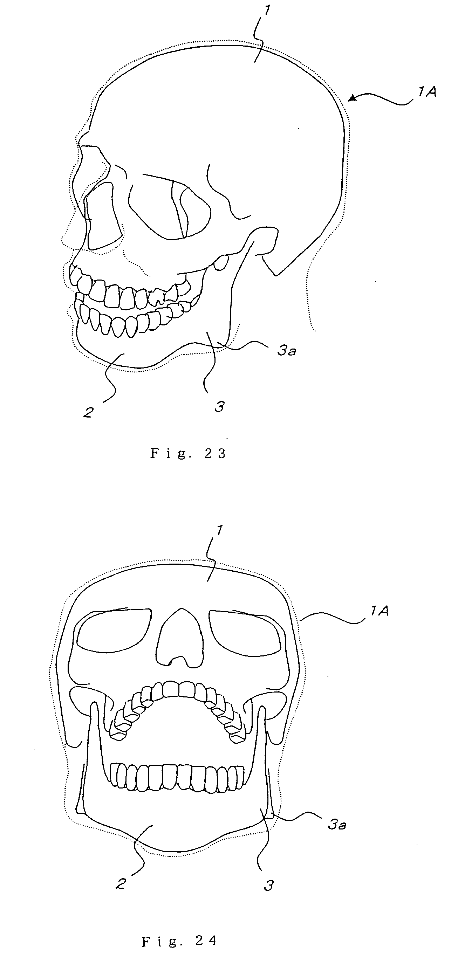

As shown in Figs. 23 and 24, when an angle of jaw 3 (likened to "branchia") of a lower jawbone 2 as a constituent of a skull 1A has overgrown because of hypertrophy of masseter muscle or when the amount of protrusion of the left angle of jaw 3 is not equal to that of the right angle of jaw 3, an operation to cut the overgrown part 3a of the lower jawbone 3 may be carried out. In Figs. 23 and 24, numeral 1 represents a head which constitutes a skull.

Many young people have a strong desire to make their face look smaller. With this background, an increasing number of people whose angles of jaw 3 are overgrown, namely "square-jawed" people, undergo an operation to remove the protruding part 3a of an angle of jaw 3 for a cosmetic purpose.

For removing the protruding part 3a of the angle of jaw 3, there are two surgical methods: one is an extraoral method in which an incision is made in the face, and the other is an intraoral method in which an incision is made inside the buccal cavity, more specifically in the area between the gingivobuccal sulcus and the buccal mucosa.

Since an operation to cut the angle of jaw 3 is often carried out for a cosmetic purpose as mentioned above, the intraoral method, which leaves no scar on the face as a result of incision, is the mainstream.

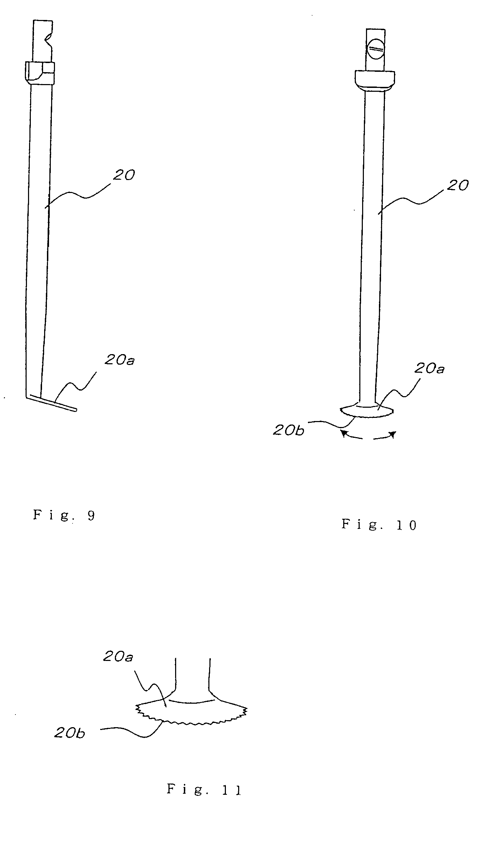

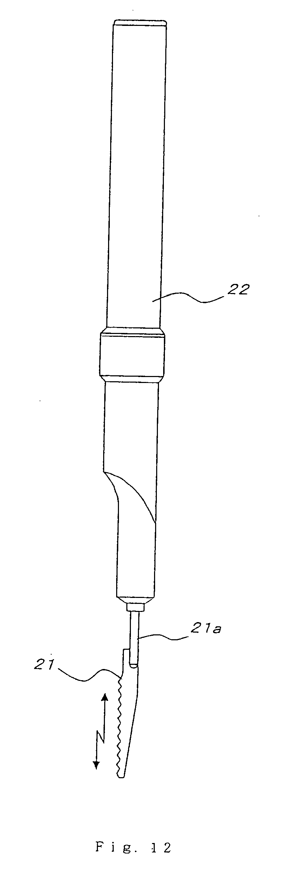

In an operation to cut the angle of jaw 3, first the protruding part 3a of the angle of jaw 3 is cut off using a bone saw 20b of an oscillating type (reciprocating type) surgical apparatus 20 as shown in Figs.9 to 11, or a bone saw 21 of an oscillating type surgical apparatus (a holder with a drive) 22 as shown in Fig.12.

Fig.11 shows details of the key part of the bone saw 20b of the oscillating type (reciprocating type) surgical apparatus 20 in enlarged form. As shown in the figure, a saw-toothed fan-shaped portion 20a reciprocates in the direction of arrow indicated in Fig.10 to cut or resect the bone.

On the other hand, the bone saw 21 of the oscillating type surgical apparatus (handpiece with a drive) 22 as shown in Fig.12 reciprocates in the direction of a shank 21a to cut or resect the bone.

When the protruding part 3a (see Fig.23) of the angle of jaw 3 (see Fig.23) is cut off as mentioned above, jags, or so-called "burrs" may be generated on the cut surface. If such burrs are left as they are, for example, relative movement between facial arteries and veins, nerves and other soft tissues, which run on the back of (under) the angle of jaw 3, and the bone cut surface might cause the bone cut surface to damage the facial arteries and veins, nerves and so on.

For this reason, it is necessary to trim the bone cut surface after cutting the angle of jaw 3.

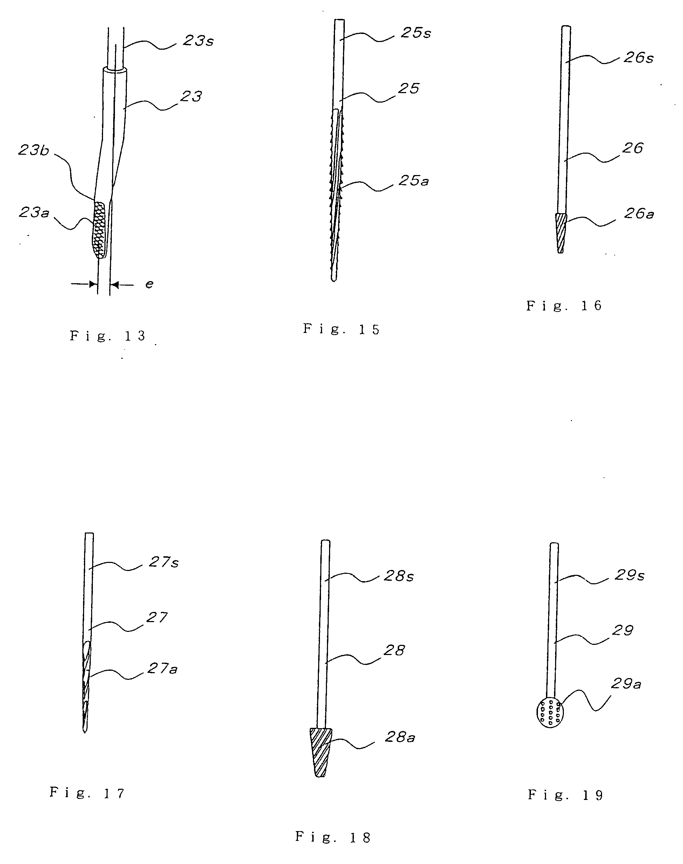

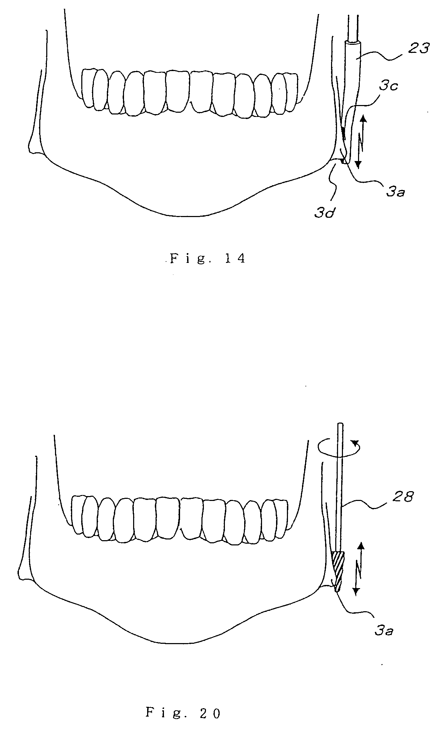

Conventionally, for such a surgical operation, apparatuses as shown in Fig.13 and Figs.15 to 19 (23, 25 to 29) have been used to trim burrs on the cut surface of the angle of jaw (Fig.14 shows how burrs are removed by the apparatus as shown in Fig.13 and Fig.20, by the apparatus as shown in Fig.18).

The apparatuses 25 to 29 as shown in Figs.15 to 19 are of the rotary type where shanks 25s to 29s and machined members 25a to 29a at their front ends rotate to trim (or polish) objects (burrs on the cut surface of the angle of jaw).

On the other hand, the apparatus 13 as shown in Fig.13 trims objects by reciprocating motion along the axial direction of a shank 23s. The apparatus 13 is designed to compensate for eccentricity (e) of a shaft 23b of a trimming file 23a with respect to the center of axle of the shank 23s.

However, the rotary apparatuses 25 to 29 are very likely to involve and damage blood vessels, nerves and other soft tissues.

Furthermore, when the conventional orthopedic apparatus 13 is used to make an incision in the area between the gingivobuccal sulcus and the buccal mucosa in accordance with the intraoral method, a surface 3c parallel to the gingivobuccal sulcus can be trimmed as shown in Fig.14, but a cut surface 3d of the angle of jaw almost perpendicular to the gingivobuccal sulcus cannot be trimmed. Consequently, it is impossible to trim (or polish) burrs on the cut surface of the angle of jaw adequately.

In order to solve the problem that it is impossible to trim the cut surface of the angle of jaw, an orthopedic apparatus 40 as shown in Figs.21 and 22 is proposed.

This orthopedic apparatus 40 has file ridges 40b inside a curved portion 40a at its front end, so that the cut surface 3d of the angle of jaw can be trimmed without damaging facial arteries and veins, nerves and so on in the vicinity of the angle of jaw 3, by reciprocating the apparatus in the direction of arrow indicated in Fig.22 with the inside of the curved portion 40a in contact with the cut surface 3d of the angle of jaw, as shown in Fig.22.

However, since the orthopedic apparatus 40 as shown in Figs.21 and 22 is large, a problem arises that a large incision must be made in a limited space, namely the area between the gingivobuccal sulcus and the buccal mucosa, in order to allow insertion of its front end or reciprocating motion in the direction of arrow in Fig.22 for trimming the cut surface of the angle of jaw.

As shown in Fig. 22 , when trimming burrs on the cut surface 3d of the angle of jaw with the orthopedic apparatus 40, a surgeon must hold the apparatus with his/her hands H1 and H2 during a trimming operation, which means that both hands H1 and H2 of the surgeon are completely unavailable or not free.

Besides, when burrs on the cut surface of the angle of jaw are to be trimmed by the orthopedic apparatus 40 as shown in Fig.21, it is impossible to perform trimming unless the patient's lower jawbone 3 is fixed properly. Therefore, another human hand H3 is needed to fix the patient's lower jawbone 3 (or the skull 1).

### Disclosure of the Invention

The present invention has been proposed in view of the above problem inherent to the conventional techniques and is intended to provide an orthopedic apparatus which enables safe trimming without damaging facial arteries/veins and nerves in the vicinity of the angle of jaw and without the need for making a large incision and allows a surgeon to perform trimming by one hand and reduces the number of persons required for an operation.

An orthopedic apparatus according to the present invention (for example, a file 10) has: a shank (11); a fan-shaped member (12) which is provided at the front end of the shank (11); and a coupling (13) which is provided at the rear end of the shank and is to be connected to a driving source, wherein the fan-shaped member (12) is inclined with respect to an extension (L) of the shank (inclination angle θ); file ridges (14) are formed on the fan-shaped member (12) 's surface (reverse side) which is oriented toward the rear end of the shank; and the shaft (11) is designed to oscillate clockwise and counterclockwise continuously (Claim 1).

According to the present invention which is embodied as mentioned above, the object (for example, a cut surface 3d of the angle of jaw 3) can be trimmed by oscillating the shank (11) and the fan-shaped portion (12) continuously with the file ridges (14) formed on the surface (reverse side) of the fan-shaped member (12) oriented toward the rear end of the shank in contact with the object to be trimmed (for example, the cut surface 3d of the angle of jaw 3).

The shank (11) is constructed so as to rotate into clockwise direction and counterclockwise direction continuously. This means that it does not rotate in one direction continuously, and arteries and veins, nerves and other soft tissues in the vicinity of the angle of jaw (3) will not get caught in the file (14); therefore there is no possibility of damaging the surrounding soft tissues and high safety is ensured.

Since the file ridges (14) are oscillated by a driving source (30) for trimming, the whole apparatus can be compact and it is unnecessary to move the apparatus handle largely by hand. Therefore, for example, when trimming the cut surface (3d) of the angle of jaw through an incision made in the area between the gingivobuccal sulcus and the buccal mucosa, the incision need not be large.

In the present invention, it is preferable that the shank (11) is straight (Claim 2).

The reason is as follows. For example, when the present invention is used to trim the cut surface (3d) of the angle of jaw in accordance with the intraoral method, if the shank (11) should be bent or curved, it would be difficult to trim the cut surface (3d) of the angle of jaw through an incision made in the area between the gingivobuccal sulcus and the buccal mucosa using the fan-shaped member (12) . Therefore, the incision must be large.

In the present invention, it is preferable to provide an attachment (32) which connects the rear end of the shank (11) and the driving source (handpiece 30) (Claim 3).

In embodying the present invention, it is preferable that the object to be trimmed is a cut surface (3d) of the angle of jaw (Claim 4).

### Brief Description of the Drawings

Fig.1 is a side view of a bone file according to an embodiment of the invention;
Fig.2 is a front view of the bone file according to the embodiment of the invention;
Fig.3 shows the key part of the file of Fig. 1 in enlarged form;
Fig.4 is an assembly drawing showing the bone file according to the embodiment of the invention which is fitted to the handpiece through an attachment, together with a fastening tool;
Fig.5 is a layout drawing separately showing the components of the bone file according to the embodiment of the invention before they are assembled onto the handpiece through the attachment, together with the fastening tool;
Fig.6 is a view of the bone file in a tilted position according to the embodiment of the invention showing how the bone file is used for an orthopedic operation;
Fig.7 is a view of the bone file in a slightly tilted position according to the embodiment of the invention showing how the bone file is used for an orthopedic operation;
Fig.8 is a view of the bone file in an almost vertical position according to the embodiment of the invention showing how the bone file is used for an orthopedic operation;
Fig.9 is a front view showing an example of a conventional bone saw;
Fig.10 is a side view of the bone saw of Fig.9;
Fig.11 is an enlarged view of details of the key part of the bone saw of Fig.9;
Fig.12 is a side view of a conventional bone saw (No.2) to which a handpiece is fitted;
Fig.13 is a perspective view showing the front end of a conventional bone file (No.1);
Fig.14 is a perspective view showing how a bone cutting operation is done using the conventional bone file (No.1);
Fig.15 is a front view of the conventional bone file (No.2);
Fig.16 is a front view of a conventional bone file (No.3) ;
Fig.17 is a front view of a conventional bone file (No. 4) ;
Fig. 18 is a front view of a conventional bone file (No. 5) ;
Fig.19 is a front view of a conventional bone file (No. 6) ;
Fig.20 is a perspective view showing how a bone cutting operation is done using the conventional bone file (No.5);
Fig.21 is a perspective view of a conventional bone file (No.7);
Fig.22 is a perspective view showing how a bone cutting operation is done using the conventional bone file (No.7);
Fig.23 is a perspective view of the constitution of the skull; and
Fig.24 is a front view of the constitution of the skull.

### Best Mode for Carrying Out the Invention

Next, an embodiment of the present invention will be described in reference to the accompanying drawings.

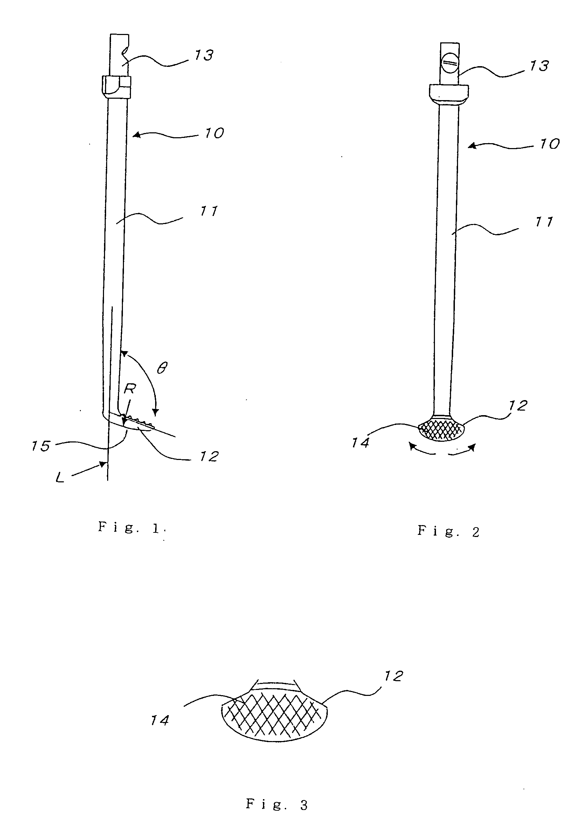

Fig.1 , Fig.2 and Fig.3 are respectively a side view and a front view showing a bone file as a separate unit according to an embodiment of the invention and an enlarged view of details of the fan-shaped member as its key part.

An orthopedic apparatus, for example a file 10, has a shank 11, a fan-shaped member 12 which is provided at the front end of the shank 11, and a coupling 13 which is provided at the rear end of the shank and is to be connected with a driving source (handpiece) 30 (stated later).

The fan-shaped member 12 is inclined by an angle of θ with respect to an axis line L and file ridges 14 are formed on the fan-shaped member 12's surface (reverse face) oriented toward the rear end of the shank.

The shank 11 is designed to oscillate clockwise and counterclockwise continuously by a driving source 30 (stated later). For a means for repeating such oscillation, a known mechanism is applicable.

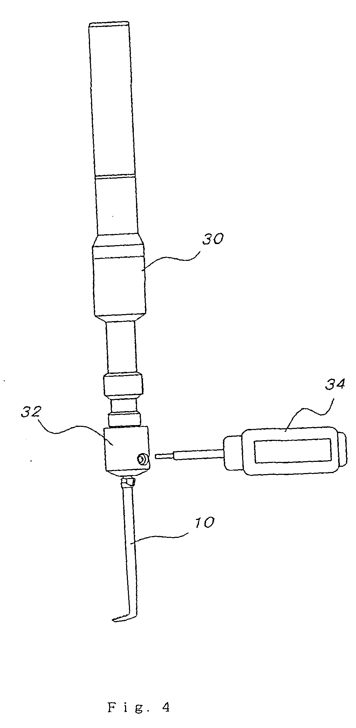

Fig.4 is an assembly drawing showing the bone file 10 according to the embodiment which is fitted to a driver (driving source: handpiece) 30 and an attachment 32, and a screw fastening tool 34 which is used to fit the bone file to the attachment 32.

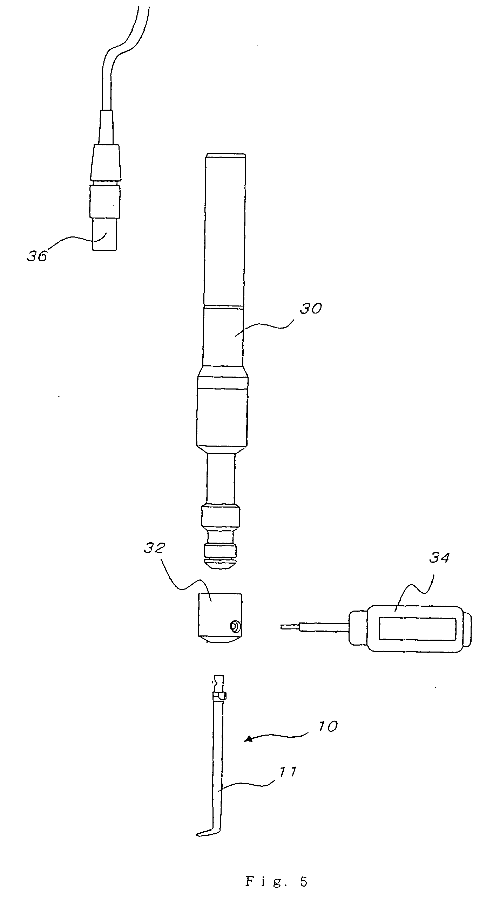

Fig.5 separately shows the attachment 32, the driver (handpiece) 30 and a cable 36 for supply of power to the driver and these components are all commercially available. The same commercial items as those used for the bone saw 20 as illustrated in Figs.9 to 11 in connection with the prior art may be used for these components.

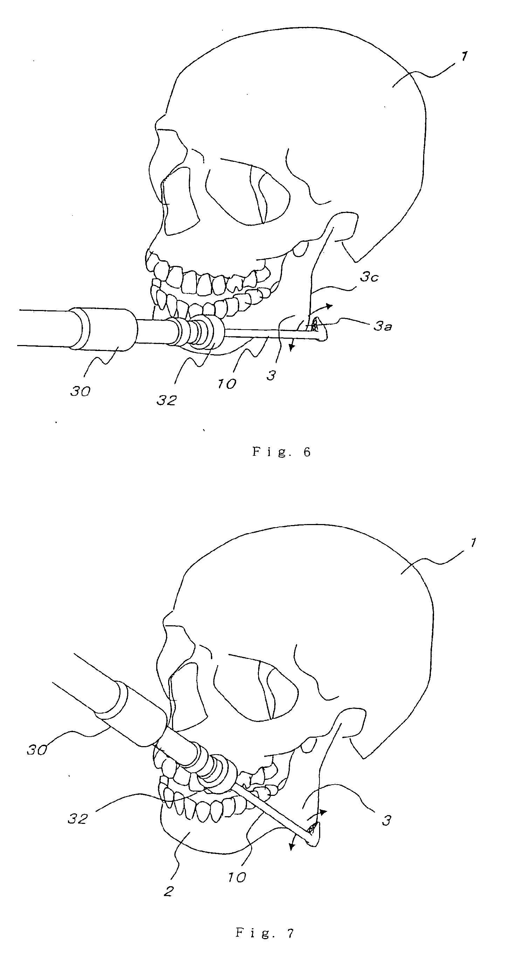

Figs.6 to 8 show how the bone file 10 is used to trim the angle of jaw 3.

Referring to Figs.6 to 8, the angle of jaw 3 is not cut off but actually the protruding part 3a of the angle of jaw 3 is cut off using the bone saw 20 as shown in Figs.9 to 11 and angular parts and/or jags (so-called "burrs") on the cut surface are trimmed and removed by oscillating the ridges 14 of the bone file 10.

As a consequence, an accident that arteries and veins and nerves in the vicinity of the angle of jaw 3 are damaged is prevented.

Figs.6 to 8 show that after an incision is made in the area between the gingivobuccal sulcus and the buccal mucosa and the angle of jaw 3 is cut, the cut surface 3d is trimmed. Here, only the skull 1A is shown and the incision made in the area between gingivobuccal sulcus and the buccal mucosa is not shown.

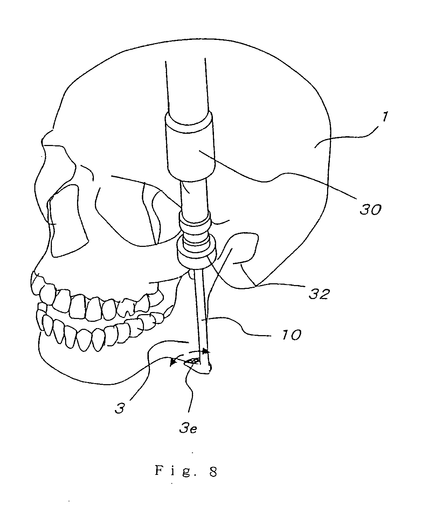

Figs.6 to 8 show that the angle of insertion of the bone file 10 differs depending on the location of burrs, or where burrs are removed. Specifically, when the posterior edge 3c of the ramus of the jawbone is to be trimmed (Fig. 6), the bone file 10 should be tilted into an almost horizontal position; and when the lower edge of the jawbone 3e is to be trimmed (Fig.8), the bone file 10 should be in an almost vertical position.

The fan-shaped member 12, on which file ridges 14 are formed, reciprocates in the direction of arrow in Figs.6 to 8 by a relatively small oscillation angle (for example, 7 degrees), so a feature common to oscillating type surgical apparatuses that the possibility of damage to soft tissues such as blood vessels and nerves is low is demonstrated.

In other words, when a rotary apparatus is used, soft tissues around an area to be trimmed get caught in the rotating apparatus, soft tissues in an extensive area might be damaged until it stops rotating; on the other hand, in case of an oscillating type apparatus, even if soft tissues should get caught, the extent of damage to them would be extremely small because of its reciprocating motion.

In addition, as apparent from Figs.6 to 8, in the bone file 10 according to the embodiment, the ridges 14 of the file only touch the cut surface. For this reason, the possibility that arteries and veins in the vicinity of the angle of jaw 3 might be damaged in trimming the cut surface of the angle of jaw 3 is very low.

Here, if the fan-shaped member 12 is too large, the apparatus might be difficult to handle. On the other hand, if it is too small, it might be difficult to perform trimming.

Furthermore, if the radial distance from the center of the fan-shaped portion to the edge should be less than 5 mm, the manufacturing cost would be higher. On the other hand, if the radial distance from the center of the fan-shaped portion to the edge should be more than 15 mm, the apparatus would be less convenient and less easy to handle in performing trimming.

It is recommended that the radial distance from the center of the fan-shaped portion to the edge be in the range from 5 to 15 mm.

The oscillation angle of the fan-shaped member 12 should be in the range from 5 to 30 degrees. If this angle should be smaller than 5 degrees, it would be difficult to trim the cut surface; and if it is larger than 30 degrees, the apparatus would oscillate too largely and be hard to handle.

The maximum reciprocating speed of the fan-shaped member 12 of the bone file 10 according to the embodiment shown in the figures is, for example, 20000 CPM (20000 reciprocations per minute).

It is preferable that such speed (reciprocating speed of the fan-shaped member 12) can be freely adjusted depending on the object to be trimmed or polished.

In the bone file 10 according to the embodiment, the surface 15 (reverse side of the file 10) of the fan-shaped member 12 on which surface 15 file ridges 14 are not formed is gently curved, so that movement in the area between the gingivabuccal sulcus and the buccal mucosa can be smooth; and also the soft tissues are not damaged when the apparatus inserted through the incision can pass through the inner soft tissues and reach the cut surface of the angle of jaw 3.

If the curvature radius of the fan-shaped member 12's surface (reverse side of the file) 15 on which file ridges 14 are not formed should be too large, that surface (reverse side of the file) 15 would be almost flat, and while the apparatus is passing through the soft tissues, resistance might occur, resulting in damage to the soft tissues

On the other hand, if the curvature radius should be too small, the surface (reverse side of the file) 15 would protrude and in an attempt to let the file ridges 14 touch the cut surface 3d of the angle of jaw, the protruding part 3a might interfere with the dermis in the area of the angle of jaw.

It is desirable to select the material of the fan-shaped member 12 on which the file ridges 14 are formed and the file coarseness appropriately depending on the object to be trimmed (or polished).

In case of the embodiment shown in the figures, it is desirable to select the material and the file coarseness which are necessary to trim the cut surface of the angle of jaw.

Though not clearly shown in the figures, for trimming the cut surface of the angle of jaw or a similar process, it is also possible to use a coarsely ridged file at the early stage of the trimming process and replace it with a finely ridged file later.

In the bone file 10 according to the embodiment, the length of the shank 11 from the attachment 32 and the driver (driving source; handpiece) 30 to the fan-shaped member 12 on which the file ridges 14 are formed should not be less than the minimum required to allow the fan-shaped member 12 to reach the cut surface of the angle of jaw 3 through an incision made in the area between the gingivobuccal sulcus and the buccal mucosa and remove burrs (perform trimming) with such members as the attachment 32 and the driver (driving source; handpiece) 30 outside the buccal cavity.

If the shank 11 should be too long, it would be hard to handle the fan-shaped member 12 at the front end of the shank, making it more difficult to remove burrs. Such a situation is undesirable.

It is recommended that the shank length be in the range from 30 to 110 mm.

It is also possible that the shank length is less than 30 mm and the handpiece 30 is inserted in the surgical wound. However, insertion of the handpiece 30 in the wound might narrow the operative field.

The shank 11 is straight and not bent or curved. If it should be bent or curved, it would be difficult to use the fan-shaped member to trim the cut surface of the angle of jaw through an incision made in the area between the gingivobuccal sulcus and the buccal mucosa and thus it would become necessary to make the incision larger.

The angle θ between the shank 11 and the fan-shaped member 12 is in the range from 90 to 180 degrees and most preferably in the range from 100 to 120 degrees.

If this angle is too large or too small, the following requirements will not be satisfied:
"such members as the attachment 32 and the driver (driving source; handpiece) 30 are located outside the buccal cavity
"the bone file 10 according to the embodiment is inserted through an incision made in the area between the gingivobuccal sulcus and the buccal mucosa and
"the file ridges 14 of the fan-shaped member 12 should touch the whole area to be trimmed for trimming the cut surface of the angle of jaw 3"

Since the bone file 10 according to the embodiment shown in the figures satisfies the above requirements, for example, in an operation to cut the angle of jaw, all necessary surgical techniques can be carried out through an incision made in the area between the gingivobuccal sulcus and the buccal mucosa and the incision can be as small as possible.

As a consequence, the burden on the patient (living body) can be minimized.

From measurements made by the inventors, it has been found that while the operation time required for conventional orthopedic surgery on the angle of jaw is approximately 1.5 hours, the use of the bone file 10 according to the embodiment shown in the figures reduces the operation time to about 1.0 hour. Since the time required for processes other than the process of trimming the angle of jaw is the same as in the conventional surgery, the operation time reduction of 0.5 hour may be thought to be the time reduction which was achieved in the orthopedic process of trimming the angle of jaw.

In addition, according to the embodiment shown in the figures, the weight of the whole apparatus (see Fig.4) including such members as the attachment 32 and the driver (driving source; handpiece) 30 is approximately 280 g and it is light enough for an ordinary adult person to handle by one hand.

Therefore, when the bone file 10 according to the embodiment shown in the figures is used, unlike the case that the orthopedic apparatus 40 as shown in Figs.21 and 22 is used, a surgeon which performs orthopedic surgery on the angle of jaw can handle the bone file 10 according to the embodiment by one hand. Hence, in the course of trimming the cut surface 3d, the surgeon can use his/her free hand to prepare for any contingency and carry out other necessary tasks (arrest of hemorrhage, suction of body fluids and others).

It should be noted that the embodiment shown in the figures is just an example and it never limits the technical scope of the present invention.

Although the embodiment shown in the figures has been described above by taking its use for trimming the cut surface of the angle of jaw as an example, the present invention may be applied to processes of trimming bone cut surfaces in other body areas. In addition, the technical scope of the present invention also covers apparatuses for polishing workpieces (objects to be machined) in various machining processes.

### Effects of the Invention

The effects of the present invention are listed below:
(1) For example, the cut surface of the angle of jaw can be trimmed safely without damaging blood vessels , nerves and other soft tissues in its vicinity.
(2) The surgical wound can be small because trimming work does not require a large incision.
(3) The operation time is shortened.
(4) Another person's help is not needed to fix the object to be trimmed such as the lower jawbone.
(5) Trimming or polishing can be performed by one hand.

## Claims

1. An orthopedic apparatus comprising:
a shank;
a fan-shaped member which is provided at the front end of the shank; and
a coupling which is provided at the rear end of the shank and is to be connected to a driving source,
wherein:
the fan-shaped member is inclined with respect to an extension of the shank,
file ridges are formed on the fan-shaped member's surface which is oriented toward the rear end of the shank, and
the shank is constructed so as to rotate into clockwise direction and counterclockwise direction continuously.

2. The orthopedic apparatus according to Claim 1, wherein the shank is straight.

3. The orthopedic apparatus according to Claim 1 or Claim 2, wherein an attachment is provided which connects the rear end of the shank and the driving source.

4. The orthopedic apparatus according to any one of Claims 1 to 3, wherein the object to be trimmed is a cut surface of an angle of jaw.
